# EUROPEAN PATENT SPECIFICATION

(11) **EP 1 502 549 B1**
(45) Date of publication and mention of the grant of the patent: **21.05.2008**
(21) Application number: 04016996.3
(22) Date of filing: 19.07.2004
(51) Int. Cl.: A61B 7/00, G06F 19/00, G06T 7/00

(54) **Medical image displaying method**
Verfahren zur Darstellung von medizinischen Bildern
Procédé d'affichage d'images médicales

(30) Priority: 23.07.2003 JP 2003200345
(43) Date of publication of application: 02.02.2005
(73) Proprietor: Konica Minolta Medical & Graphic, Inc., Hino-shi, Tokyo 191-8511 (JP)
(72) Inventor: Wada, Yasunori, Hachioji-shi, Tokyo 192-8505 (JP); Kasai, Satoshi, Hachioji-shi, Tokyo 192-8505 (JP)
(74) Representative: Henkel, Feiler & Hänzel

(56) References cited:
- DE-A- 4 030 448
- US-A1- 2003 130 588
- HENNEBERG S ET AL: "Remote auscultatory patient monitoring during magnetic resonance imaging" J CLIN MONIT; JOURNAL OF CLINICAL MONITORING JAN 1992, vol. 8, no. 1, January 1992 (1992-01), pages 37-43, XP008038094
- KOMPIS M ET AL: "Acoustic Imaging of the Human Chest" CHEST, THE COLLEGE, CHICAGO, IL, US, vol. 120, no. 4, October 2001 (2001-10), pages 1309-1321, XP002237833 ISSN: 0012-3692
- KUMAR A SUKESH ET AL: "Relevance of the development of medical workstation for diagnosis and treatment" MODELL MEAS CONTROL C; MODELLING, MEASUREMENT AND CONTROL C 1999 AMSE PRESS, TASSIN-LA-DEMI-LUNE, FRANCE, vol. 60, no. 2, 1999, pages 54-66, XP008038089
- ROSS ROBERT M: "The clinical diagnosis of asbestosis in this century requires more than a chest radiograph." CHEST, vol. 124, no. 3, September 2003 (2003-09), pages 1120-1128, XP008038303 ISSN: 0012-3692

## Description

### FIELD OF THE INVENTION

The present invention relates to a medical image displaying method, program, and recoding medium for creating a medical image used for image diagnosis.

### BACKGROUND OF THE INVENTION

In the field of medical care, various forms of diagnosis have been practiced, including image diagnosis using such a medical image as a radiographic image and examination with a stethoscope.

In recent years, image diagnosis has reached a high degree of sophistication with the development of medical technology, with the result that a wide variety of medical images based on an X-ray radiography and CT technology are now available.

In many cases, very useful diagnostic information can be obtained from the biological sound detected by a stethoscope, and therefore examination with a stethoscope has been occupying a crucial position essential to diagnosis. For example, interstitial pneumonia in an early stage hardly exhibits any change in X-ray radiographic image, or shows only a very light shadow even if there is any change. By contrast, the biological sound detected by a stethoscope provides clear diagnostic information indicative of interstitial pneumonia in some cases.

Particularly in recent years, U.S. Patent No. 6,139,505, for example, discloses a technology for detecting a source of the biological sound (hereinafter referred to as "sound source detecting method"). Further, the Official Gazette of Japanese Patent Tokkai 2001-505085, for example, discloses a technology for automatic identification of the type of respiratory sounds using a stethoscope (hereinafter referred to as "respiratory sound type identification method"). These examples reveal that an improved support is being provided for examination with a stethoscope.

The aforementioned "respiratory sound" is a generic term to refer to the sound in the pectoral region, caused by respiration and detected by a stethoscope. It can be broadly classified into two types: one is a breath sound as a sound of air flow occurring in the respiratory tract and alveoli pulmonis, and the other is an adventitious sound (e.g. a discontinuous rale which is an abnormal sound).

With an increase in the types of medical images, a radiologist is required to read a lot of medical images for diagnosis, and therefore time of reading one sheet of medical image tends to be reduced on average. As a result, conventional simple X-ray radiographs, for example, are often read by a clinician in charge, instead of an expert radiologist having been trained for expertise and skill. This may cause an unforeseen accident such as oversight of the lesion or a diagnostic error. Under such conditions, development of technologies for improving precision in image diagnosis is receiving widespread attention.

The object of the present invention is to improve the precision in image diagnosis using a medical image including a radiographic image.

US2003/0130588A1 discloses a system and a method for analysing respiratory tract sounds. The system comprises a plurality of transducers that are placed on the individual's skin over the thorax. A signal analysis module analyses sound signals recorded by the transducers and produces a first analysis product. A display device displays an image based upon the first analysis product. The image includes a pattern with an overall shape corresponding to that of the respiratory system. Regions in the image that are suspected of having a pathological condition may be indicated.

### SUMMARY OF THE INVENTION

According to the present invention the above object is achieved by a medical image displaying method according to claim 1, or 14, by a computer program according to claim 27 and by a recording medium according to claim 28 comprising the computer program. The dependent claims are directed to further advantageous aspects of the present invention.

To achieve the aforementioned object, the present invention provides:
(1) A medical image display method, for creating and displaying a medical image to be read at the time of image diagnosis, comprising:
   a radiographed image data acquisition step for acquiring the image data containing a predetermined site of a patient radiographed thereon;
   a biological sound detection data acquisition step for acquiring data on detected biological sounds produced from the aforementioned radiographed site;
   a sound source identification step for identifying the sound source of the biological sound, based on the data on detected biological sounds; and
   an image display step for displaying the aforementioned identified sound source superimposed over the aforementioned radiographed site.
(2) A medical image display method, for creating and displaying a medical image to be read at the time of image diagnosis, comprising:
   a radiographed image data acquisition step for acquiring the image data containing a predetermined site of a patient radiographed thereon;
   a biological sound detection data acquisition step for acquiring the data on detected biological sounds produced from the aforementioned radiographed site;
   a sound source identification step for identifying the sound source of the biological sound, based on the aforementioned acquired data on detected biological sounds; and
   an image display step for parallel display of the aforementioned identified sound source and the aforementioned radiographed image.
(3) In the medical image display method described in (1) or (2), it is preferred that the aforementioned data on detected biological sounds and/or radiographed image should be acquired via the data line.
(4) In the medical image display method described in any one of (1), (2) and (3), it is preferred that the aforementioned radiographed image data should be the image data captured by X-rays, CT (Computed Tomography), MRI (Magnetic Resonance Imaging), magnetic instrumentation, biological electric signal, ultrasonic waves or light.
(5) In the medical image display method described in any one of (1), (2), (3) and (4), it is preferred that the sound source of the biological sound should be identified according to the position of the microphone when collecting the aforementioned biological sound.
(6) In the medical image display method described in any one of (1), (2), (3), (4) and (5), it is preferred that the aforementioned biological sound detection data acquisition step and sound source identification step should be repeated alternately until an instruction for termination is inputted.
(7) In the medical image display method described in any one of (1), (2), (3), (4), (5) and (6), it is preferred that the image of a predetermined area including the sound source of the aforementioned biological sound should be extracted from the radiographed image in the aforementioned image display step, and the extracted image should be displayed in an enlarged form.
(8) In the medical image display method described in any one of (1), (2), (3), (4), (5), (6) and (7), it is preferred that the aforementioned image displayed in an enlarged form should lie one on top of the area of the radiographed image including the aforementioned sound source.
(9) In the medical image display method described in any one of (1), (2), (3), (4), (5), (6), (7) and (8), it is preferred that aforementioned image displayed in an enlarged form should be displayed in the form associated with the area of the radiographed image including the aforementioned sound source in the aforementioned image display step.
(10) In the medical image display method described in any one of (1), (2), (3), (4), (5), (6), (7), (8) and (9), it is preferred that the type of the biological sound should be identified from the data on detected biological sounds in the aforementioned image display step, and the aforementioned sound source should be displayed based on the form of display conforming to the type of the identified biological sound.
(11) In the medical image display method described in (10), it is preferred that the type of the sound source whose position is displayed on the image should be displayed for the image to be displayed in an enlarged form.
(12) In the medical image display method described in any one of (1), (2), (3), (4), (5), (6), (7), (8), (9), (10) and (11), it is preferred that, when the data on detected biological sound is related to the data on the respiratory sound, the sound source position of the respiratory sound should be adjusted in response to either expiration or inhalation in the display step, and the sound source position subsequent to this adjustment should be displayed.
(13) In the medical image display method described in (12), it is preferred that the information for identifying expiration from inhalation should be included in the data on detected biological sound.
(14) In the medical image display method described in (12) or (13), it is preferred that the aforementioned sound source position should be adjusted based on the expansion and contraction of lungs at the time of respiration.

Thus, the position of the biological sound generating source can be easily identified synchronously with reading of the medical image such as an X-ray image. Further, many other pieces of information (e.g. the image showing the source of the biological sound displayed on top of, or in parallel with, the radiographed image, or the radiographed image where a predetermined area including the sound source is displayed in an enlarge form) other than radiographed image can be displayed on the medical image; and hence this arrangement ensures improved concentration of the doctor reading the image and minimizes the possibility of overlooking a lesion or committing a diagnostic error. To put it another way, this arrangement improves precision in image diagnosis.

### BRIEF DESCRIPTION OF THE DRAWINGS

Fig. 1 is a diagram representing the configuration of a medical image display system conforming to the present invention;
Fig. 2 is a flowchart representing an example of a medical image display method conforming to the present invention; and
Fig. 3 is a drawing representing an example of the image display created by a medical image display method conforming to the present invention.

### DETAILED DESCRIPTION OF THE PREFERRED EMBODIMENT

The following describes a medical image display system 100 conforming to the present invention with reference to the drawings:

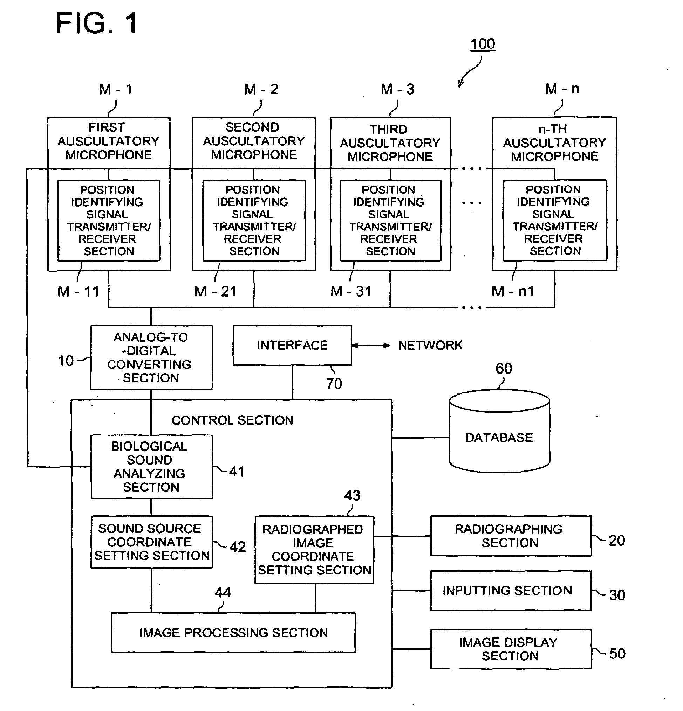

As shown in Fig. 1, the medical image display system 100 is equipped with a first auscultatory microphone M-1 through an n-th auscultatory microphone M-n, an analog-to-digital converting section 10, a radiographing section 20, an inputting section 30, a control section 40, an image display section 50, a database 60 and an interface 70.

The first auscultatory microphone M-1 through n-th auscultatory microphone M-n are attached to the body of a patient. It captures a biological sound such as a respiratory sound, converts the biological sound into the biological sound detection signal of analog format and outputs it to the analog-to-digital converting section 10.

The first auscultatory microphone M-1 through n-th auscultatory microphone M-n are equipped with a position identifying signal transmitter/receiver section M-11 through a position identifying signal transmitter/receiver section M-n1, respectively. The position identifying signal transmitter/receiver section M-11 through a position identifying signal transmitter/receiver section M-n1 transmit and receive the position identifying signals for identifying the installation positions of the first auscultatory microphone M-1 through n-th auscultatory microphone M-n. The following description assumes that position identifying signals are sound waves, and the first auscultatory microphone M-1 through n-th auscultatory microphone M-n transmit the sound waves of inherent frequency as position identifying signals.

In the analog-to-digital converting section 10, the biological sound detecting signal inputted from the first auscultatory microphone M-1 through n-th auscultatory microphone M-n is sampled at a predetermined sampling frequently, and is converted into the digital data on detected biological sound. Then the data on detected biological sound is sent to a biological sound analyzing section 41.

The radiographing section 20 creates a medical image by radiographing a subject using X-rays, CT (Computed Tomography), MRI (Magnetic Resonance Imaging), magnetic instrumentation, biological electric signal, ultrasonic waves or light.

The inputting section 30 is equipped with a keyboard and various pointing devices (mouse, touch panel, etc.), and is used to apply various types of instructions to the control section 40.

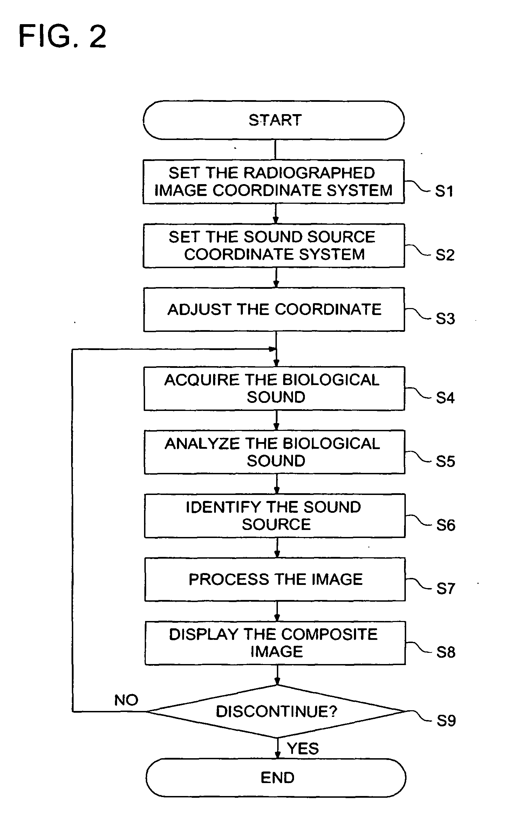

The control section 40 is a biological sound analyzing section 41, a sound source coordinate setting section 42, a radiographed image coordinate setting section 43, an image processing section 44, a CPU (Central Processor Unit) (not illustrated), a built-in memory, etc. to control the medical image display system 100 in a comprehensive manner. The aforementioned memory contains various programs (including the medical image creating program for applying processing of medical image creation, as shown in the flowchart of Fig. 2) to be implemented by the control section 40.

The CPU reads various programs including the medical image display program stored in the aforementioned memory and provides centralized control of the operations of various sections according to the processing program. The CPU also executes various types of processing according to various processing program, and stores the result of processing in the aforementioned memory. At the same time, it allows the result of processing to be displayed on the image display section 50, and sends the stored result of processing to a predetermined destination where this result is stored.

Further, the aforementioned memory has a recording medium (not illustrated) for storing programs, data and others in advance. This recording medium is composed of a magnetic/optical recording medium or a semiconductor memory. This recording medium is fixed on the aforementioned memory or is mounted removably. This recording medium stores various processing programs including the medical image display program, as well as data processed by various processing programs. These various processing programs are stored in the form of a program code that can be read. The CPU sequentially executes operations according to the program code.

The aforementioned medical image creating program may be stored in various recording media such as a CD (Compact Disk) in advance. In this case, the medical image display system 100 is equipped with a driver designed specifically for free reading and writing of data into the recording medium.

Using the biological sound analyzing section 41 through the image processing section 44, the control section 40 applies processing of medical image creation as shown in the flowchart of Fig. 2. This processing of medical image creation may be implemented by the aforementioned medical image creating program or by hardware.

The image display section 50 is equipped with such a display apparatus as an LCD (liquid crystal display) and a CRT (cathode ray tube), and displays various types of image data inputted from the control section 40.

The database 60 stores the data on detected biological sound in the form associated with the data on a patient name and data creation date/time. It also stores the biological sound identification code representing the type of the biological sound. This data on detected biological sound may be classified according to the type of biological sound by the biological sound analyzing section 41. In this case, each of the classified data item on detected biological sound is associated with the biological sound identification code indicating the type of biological sound, in addition to the patient name and data creation date/time.

The database 60 may contain the radiographed image data captured by the radiographing section 20, in the form associated with the data showing the patient name and date/time of radiographing. Alternatively, each of the samples of the data on detected biological sound and radiographed image data may be stored therein.

The interface 70 has an interface for sending or receiving data via the network such as LAN (Local Area Network).

The following describes the details of processing of medical image creation with reference to Fig. 2. The following description of the operation assumes that the program for performing various processing described in the following flowchart is stored in the memory arranged in the control section 40 having a CPU, in the form of a program code readable to a computer, where the CPU sequentially performs the operations according to this program code.

Firstly, for the radiographed image captured by the radiographing section 20, the radiographed image coordinate setting section 43 sets two-dimensional or three-dimensional coordinate (hereinafter referred to as "radiographed image coordinate system"), in response to the dimension (two-dimensional or three-dimensional image) of this radiographed image (Step S1).

In this case, the radiographed image is not restricted to the one captured by the radiographing section 20; it may be the radiographed image data stored in the database 60 or the radiographed image data acquired via the interface 70. In any case, the radiographed image as electronic data is acquired via the data line (network line such as LAN, serial or USB line, etc.).

Of the first auscultatory microphone M-1 through n-th auscultatory microphone M-n, the first auscultatory microphone M-1 and second auscultatory microphone M-2 in the case of a two-dimensional image (or the first auscultatory microphone M-1 through third auscultatory microphone M-3 in the case of a three-dimensional image) are assumed to be radiographed in the radiographed image; this is because the position of the microphone in the image will be identified later. In this case, at least two auscultatory microphones are required to identify the position of the microphone in the image, in the case of a two-dimensional image. For a three-dimensional image, at least three microphones are required. For simplicity, the radiographed image is assumed as a three-dimensional image in the following description.

Then the sound source coordinate setting section 42 sets the coordinate (hereinafter referred to as "sound source coordinate system") to identify the sound source of the biological sound detected by the first auscultatory microphone M-1 (Step S2). The dimension of the sound source coordinate system is the same as that of the radiographed image coordinate system.

In this case, the sound source coordinate setting section 42 allows each of the position identifying signal transmitter/receiver section M-11 through a position identifying signal transmitter/receiver section M-n1 to transmit the sound waves of inherent frequencies (position identification signals). The relative position (including the distance) of the first auscultatory microphone M-1 through n-th auscultatory microphone M-n is calculated, based on the difference in phase between the sound waves (position identification signals) received from the position identifying signal transmitter/receiver section M-11 through a position identifying signal transmitter/receiver section M-n1, and the sound waves (position identification signals) sent to the position identifying signal transmitter/receiver section M-11. According to the result of this calculation, the position coordinates of the first auscultatory microphone M-1 through n-th auscultatory microphone M-n in the sound source coordinate system is calculated.

The image processing section 44 adjusts the radiographed image coordinate system and sound source coordinate system set in each of the aforementioned Steps S1 and S2, in the following manner, for example:

The image processing section 44 applies coordinate transformation (including scale transform, rotational transform and parallel translation) to the sound source coordinate system in such a way that the position coordinate of the first auscultatory microphone M-1 through third auscultatory microphone M-3 in the aforementioned radiographed image coordinate system will agree with the position coordinate of the first auscultatory microphone M-1 through third auscultatory microphone M-3 in the sound source coordinate system. This arrangement allows the position coordinates of the first auscultatory microphone M-1 through third auscultatory microphone M-3 to be the same between the aforementioned radiographed image coordinate system and sound source coordinate system.

Upon receipt of the data on detected biological sound from the analog-to-digital converting section 10 (Step S4), the biological sound analyzing section 41 identifies the type of the data on detected biological sound according to the type, using the aforementioned method of identifying biological sound by type. At the same time, based on the result of this identification, the biological sound analyzing section 41 assigns the biological sound identifying code showing the type of biological sound, to the identified data on detected biological sound (Step S5).

When the biological sound is a respiratory sound, the biological sound analyzing section 41 can distinguish between expiration and inhalation from the data on detected biological sound, and can further classify the data on detected biological sound into data corresponding to expiration and that corresponding to inhalation. In this case, means may also be provided to ensure that the distinction between expiration and inhalation is made, for example, based on the information on the expiration and exhalation inputted through the inputting section 30 when the operator collects the respiratory sound of the patient. Alternatively, it is also possible to make such arrangements that this distinction is made automatically by comparison with the waveform data inherent to expiration and inhalation (e.g. by comparison by statistics process).

Using the aforementioned sound source detecting method, the sound source coordinate setting section 42 identifies the source of each biological sound corresponding to the data on detected biological sound (Step S6). In this case, the sound source is identified as the position relative to the first auscultatory microphone M-1 through n-th auscultatory microphone M-n.

After that, based on the position of the sound source identified as shown above, the sound source coordinate setting section 42 calculates the coordinate of each sound source position in the sound source coordinate system subsequent to the aforementioned transformation.

In the manner described above, corresponding biological sound identifying codes and expiration/inhalation identification information are added to the data sets on detected biological sound classified according to the type of biological sound and expiration/inhalation.

Then the radiographed image coordinate setting section 43 applies the following image processing to the radiographed image (Step S7).

In the radiographed image coordinate setting section 43, the character display image for displaying the biological sound character indicating the biological sound source is superimposed on the radiographed image (hereinafter referred to as "composite image") in the position coordinate of each sound source. This biological sound character may have its shape and color different for each type of biological sound or may be shown in blinking display.

In the radiographed image coordinate setting section 43, the radiographed image having been superimposed as described above is displayed on the image display section 50 (Step S8).

The image processing section 44 is capable of displaying the character display image and radiographed image in separate display frames in parallel, without the need of superimposing them.

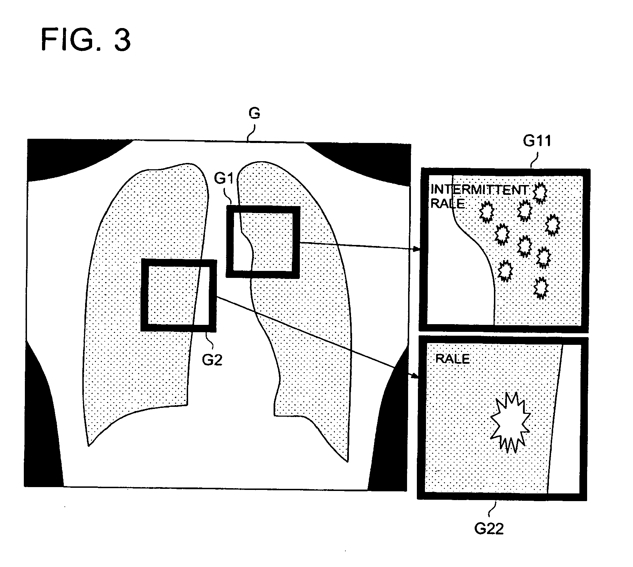

Further, the sound source coordinate setting section 42 is capable of determining whether the biological sound sources are closely packed or not. If they are closely packed, the image processing section 44 extracts the image in the coordinate area including the closely packed area, from the character display image, and enlarges it at a predetermined magnification factor (hereinafter referred to as "sound source packed image"). It is also possible to display this sound source packed image on the image display section 50 so that it will not be superimposed on the composite image (radiographed image or character display image) (Fig. 3). In this case, whether the biological sound sources are closely packed or not can be determined by checking, for example, if five or more sound sources are present in a predetermined coordinate area or not, or if ten or more sound sources are present in a predetermined coordinate area or not. This number can be determined as required by the operator.

Further, if the biological sound is a respiratory sound, the image processing section 44 is capable of enlarging or reducing in size the character display image at a predetermined magnification factor (or based on preset expansion/contraction model of the lungs) in response to either expiration or inhalation. This arrangement allows the position of the sound source to be adjusted in response to the changes in the shape of the lungs (contraction in the vertical direction for expiration, and expansion in the vertical direction for inhalation) at the time of expiration and inhalation. In this case, the sound source packed image is extracted from the composite image based on the character display image enlarged or reduced in response to expiration or inhalation as described above.

Fig. 3 shows the composite image G (radiographed image of lungs) displayed on the display screen of the image display section 50, as well as sound source packed images G11 and G22.

As shown in Fig. 3, when the packed sound sources belong to a special type of the biological sound (this is identified by the sound source coordinate setting section 42, based on the biological sound identification code), the image processing section 44 is capable of displaying the name showing the type of the biological sound (e.g. "discontinuous rale" and "rale") on the sound source packed image. In this case, the name information is associated with the biological sound identification code and is stored in the memory built in the control section 40 or the database 60.

Further, the image processing section 44 allows the image areas G1 and G2 in the composite image G corresponding to sound source packed images G11 and G22 displayed in enlarged form as described above, to be displayed in the composite image, using the arrow mark (dotted line or other objects). This arrangement provides easy identification of the area of the composite image corresponding to the sound source packed image displayed in enlarged form. It is also possible to make such arrangements that the aforementioned correspondence is identified by adjusting the frame display colors, without using such an object as an arrow.

Going back to the flowchart of Fig. 2, after Step S8, evaluation is made to determine whether the instruction to show that continued display of a composite image or others will not be made (instruction of termination) has been inputted or not (Step S9). Processing of the aforementioned Steps S4 through S9 is repeated until this instruction of termination is given, and generation and extinction of the biological sound are displayed in chronological order.

As described above, the medical image display system 100 displays the medical image such as a radiographic image superimposed on (or separately from) the position of the biological sound source acquired from the site of radiographing the medical image, through the first auscultatory microphone M-1 through n-th auscultatory microphone M-n.

Thus, the site of producing biological sound can be easily identified at the timed interval of reading a medical image such as an X-ray image. Further, much information other than the radiographed image (e.g. image showing the biological sound source and sound source packed image) can be indicated on the medical image. This enhances the doctor's power of concentration upon reading the image, thereby reducing the possibility of overlooking the lesion or committing a diagnostic error. To put it another way, this arrangement improves the precision in image diagnosis.

It should be noted that the description in the present embodiment is concerned with only an example of the medical image display method of the present invention, without being restricted thereto. The detailed configuration and operation of the medical image display system 100 in the present embodiment can be modified as required, without departing from the scope of the present invention as claimed.

In the present embodiment, the major examples are given for the cases where the biological sound is a respiratory sound. The present invention is applied to cardiac sounds or other sounds without being restricted to respiratory sounds.

Further, means may also be provided to ensure that, only when the biological sound identified by the biological sound analyzing section 41 belongs to abnormal noise, the position of the aforementioned biological sound source is displayed. The abnormal noise in this case refers to the sound that is not produced from a body not affected by illness. For example, when the biological sound is respiratory sound, such an adventitious sound as intermittent rale or rale is included in the abnormal noise.

The present invention is applicable to animals as well to humans. Especially in the case of an animal, there is no accumulation of varied type of information as compared with the case of humans, and hence reading of a medical image is accompanied with difficulties. However, the medical image display system 100 provides sufficient effects to the animal where there is a shortage of information for reading of an image.

Means may also be provided to ensure that the sound source coordinate system by the first auscultatory microphone M-1 or the like can be set using the clothing where the first auscultatory microphone M-1 through n-th auscultatory microphone M-n are fixed in advance. In this case, there is no need of measuring the positions of the first auscultatory microphone M-1 through n-th auscultatory microphone M-n every time, thereby enhancing convenience for a user.

It is also possible to make such arrangements that the radiographed image coordinate system and sound source coordinate system are adjusted by regulating the position of the heart in the sound source coordinate system identified by using the cardiac sound detected by the first auscultatory microphone M-1 and the like, to the position of the heart in the radiographed image.

### EFFECTS OF THE INVENTION

The present invention provides easy identification of a biological sound generating source synchronously with reading of the medical image such as an X-ray image. Further, many other pieces of information (e.g. the image showing the source of the biological sound displayed on top of, or in parallel with, the radiographed image, or the radiographed image where a predetermined area including the sound source is displayed in an enlarge form) other than radiographed image can be displayed on the medical image; and hence this arrangement ensures improved concentration of the doctor reading the image and minimizes the possibility of overlooking a lesion or committing a diagnostic error. To put it another way, this arrangement improves precision in image diagnosis.

## Claims

1. A medical image displaying method, for displaying a medical image (G) to be read at the time of image diagnosis, comprising the steps of:
a radiographed image data acquiring step for acquiring the radiographed image data (20, 60) of a predetermined part of an object to be radiographed;
a biological sound-data detecting step (54) for detecting the biological sound-data from the object;
a sound source position specifying step (55) for specifying the sound source position where the biological sound-data is detected from; and
an image displaying step (58) for displaying the medical image including a sound source position image (G1, G2) related to the specified sound source position and the radiographed image (G) related to the radiographed image data so that the sound source position image is superimposed on a part of the radiographed image corresponding with the specified sound source position.

2. The method of claim 1, wherein at least one of the radiographed image data and the biological sound-data is acquired from a database via a data line.

3. The method of claim 1, wherein the radiographed image data is the image data captured by at least one of X-rays, CT, MRI, magnetic instrumentation, biological electric signal, ultrasonic wave and light.

4. The method of claim 1, wherein the sound source position is specified according to the position of the microphone when collecting the biological sound.

5. The method of claim 1, wherein the biological sound-data detecting step, the sound source position specifying step and the image displaying step are repeated alternately until an instruction for termination is inputted.

6. The method of claim 1, wherein the image displaying step further comprises the steps of:
an image extracting step for extracting the image of a predetermined area including the sound source position from the radiographed image; and
an enlarging step for enlarging the extracted image to displaying the extracted image in an enlarged form.

7. The method of claim 6, wherein the extracted image in an enlarged form is displayed in a separate display frame.

8. The method of claim 6, wherein the extracted image in an enlarged form is according to the area of the radiographed image including the sound source.

9. The method of claim 1, wherein the image displaying step further comprises the steps of:
an identifying step for identifying the type of the biological sound from the detected biological sound-data to display the sound source position based on the form of display corresponding to the identified type of the biological sound.

10. The method of claim 6, wherein the sound source position in extracted image in an enlarged form is displayed with the type of the sound source.

11. The method of claim 1, when the detected biological sound-data is related to the data on the respiratory sound, the sound source position of the respiratory sound is adjusted in response to either expiration or inhalation in the image displaying step, and the sound source position subsequent to this adjustment is displayed.

12. The method of claim 11, wherein the information for identifying expiration from inhalation is included in the data on detected biological sound-data.

13. The method of claim 11, wherein the sound source position is adjusted based on the expansion and contraction of lungs at the time of respiration.

14. A medical image displaying method, for displaying a medical image (G) to be read at the time of image diagnosis, comprising the steps of:
a radiographed image data acquiring step for acquiring the radiographed image data (20, 60) of a predetermined part of an object to be radiographed;
a biological sound-data detecting step (54) for detecting the biological sound-data from the object;
a sound source position specifying step (55) for specifying the sound source position where the biological sound-data is detected from; and
an image displaying step (58) for displaying a sound source position image (G11, G22) related to the specified sound source position and the radiographed image (G) elated to the radiographed image data side-by-side.

15. The method of claim 14, wherein at least one of the radiographed image data and the biological sound-data is acquired from a database via a data line.

16. The method of claim 14, wherein the radiographed image data is the image data captured by at least one of X-rays, CT, MRI, magnetic instrumentation, biological electric signal, ultrasonic wave and light.

17. The method of claim 14, wherein the sound source position is specified according to the position of the microphone when collecting the biological sound.

18. The method of claim 14, wherein the biological sound-data detecting step, the sound source position specifying step and the image displaying step are repeated alternately until an instruction for termination is inputted.

19. The method of claim 14, wherein the image displaying step further comprises the steps of:
an image extracting step for extracting the image of a predetermined area including the sound source position from the radiographed image; and
an enlarging step for enlarging the extracted image to displaying the extracted image in an enlarged form.

20. The method of claim 19, wherein the extracted image in an enlarged form is displayed in a separate display frame.

21. The method of claim 19, wherein the extracted image in an enlarged form is according to the area of the radiographed image including the sound source.

22. The method of claim 14, wherein the image displaying step further comprises the steps of:
an identifying step for identifying the type of the biological sound from the detected biological sound-data to display the sound source position based on the form of display corresponding to the identified type of the biological sound.

23. The method of claim 19, wherein the sound source position in extracted image in an enlarged form is displayed with the type of the sound source.

24. The method of claim 14, when the detected biological sound-data is related to the data on the respiratory sound, the sound source position of the respiratory sound is adjusted in response to either expiration or inhalation in the image displaying step, and the sound source position subsequent to this adjustment is displayed.

25. The method of claim 24, wherein the information for identifying expiration from inhalation is included in the data on detected biological sound-data.

26. The method of claim 24, wherein the sound source position is adjusted based on the expansion and contraction of lungs at the time of respiration.

27. A computer program to control a computer to function as a medical image display, wherein the computer program causes the medical image display to perform the method of any of claims 1 to 26.

28. A recording medium, which comprises a computer program of claim 27.

## Patentansprüche

1. Medizinisches Bildanzeigeverfahren zum Anzeigen eines medizinischen Bildes (G), das zur Zeit der Bilddiagnose zu lesen ist, mit den folgenden Schritten:
einem Erfassungsschritt für Radiogramm-Bilddaten zum Erfassen der Radiogramm-Bilddaten (20, 60) eines vorbestimmten Teils mittels eines Radiogramm zu untersuchenden Objekts;
einem Detektierschritt für biologische Schalldaten (54) zum Detektieren der biologischen Schalldaten von dem Objekt;
einem Spezifizierschritt für die Schallquellenposition (55) zum Spezifizieren der Schallquellenposition, von der die biologischen Schalldaten detektiert werden; und
einem Bildanzeigeschritt (58) zum Anzeigen des medizinischen Bildes mit einem Schallquellenpositionsbild (G1, G2), das sich auf die spezifizierte Schallquellenposition bezieht, und des Radiogramm-Bildes (G), das sich auf die Radiogramm-Bilddaten bezieht, sodass das Schallquellenpositionsbild über einen Teil des Radiogramm-Bildes überlagert wird, das der spezifizierten Schallquellenposition entspricht.

2. Verfahren gemäß Anspruch 1, bei dem mindestens die Radiogramm-Bilddaten und/oder die biologischen Schalldaten über eine Datenleitung erfasst werden.

3. Verfahren gemäß Anspruch 1, bei dem die Radiogramm-Bilddaten die Bilddaten sind, die durch mindestens eines der Folgenden eingefangen werden: Röntgenstrahlen, CT, MRI, magnetische Instrumentierung, biologisches elektrisches Signal, Ultraschallwelle und Licht.

4. Verfahren gemäß Anspruch 1, bei dem die Schallquellenposition gemäß der Position des Mikrophons spezifiziert wird, wenn der biologische Schall gesammelt wird.

5. Verfahren gemäß Anspruch 1, bei dem der Detektierschritt für biologische Schalldaten, der Spezifierschritt für die Schallquellenposition und der Bildanzeigeschritt abwechselnd wiederholt werden, bis eine Anweisung zur Beendigung eingegeben wird.

6. Verfahren gemäß Anspruch 1, bei dem der Bildanzeigeschritt ferner die folgenden Schritte umfasst:
einen Bildextrahierschritt zum Extrahieren des Bildes eines vorbestimmten Bereichs, der die Schallquellenposition des Radiogramm-Bildes einschließt; und
einen Vergrößerungsschritt zum Vergrößern des extrahierten Bildes, um das extrahierte Bild in einer vergrößerten Form anzuzeigen.

7. Verfahren gemäß Anspruch 6, bei dem das extrahierte Bild in einer vergrößerten Form in einem getrennten Anzeige-Frame angezeigt wird.

8. Verfahren gemäß Anspruch 6, bei dem das extrahierte Bild in einer vergrößerten Form gemäß der Fläche des Radiogramm-Bildes ist, das die Schallquelle einschließt.

9. Verfahren gemäß Anspruch 1, bei dem der Bildanzeigeschritt ferner den folgenden Schritt umfasst:
einen Identifizierschritt zum Identifizieren des Typs des biologischen Schalls aus den detektierten biologischen Schalldaten, um die Schallquellenposition basierend auf der Form der Anzeige anzuzeigen, die dem identifizierten Typ des biologischen Schalls entspricht.

10. Verfahren gemäß Anspruch 6, bei dem die Schallquellenposition in dem extrahierten Bild in einer vergrößerten Form mit dem Typ der Schallquelle angezeigt wird.

11. Verfahren gemäß Anspruch 1, wenn die detektierten biologischen Schalldaten in Beziehung zu den Daten über den respiratorischen Schall stehen, die Schallquellenposition des respiratorischen Schalls als Antwort entweder auf Exspiration oder Inhalation bei dem Bildanzeigeschritt eingestellt und die Schallquellenposition im Anschluss an diese Einstellung angezeigt wird.

12. Verfahren gemäß Anspruch 11, bei dem die Information zum Identifizieren der Exspiration gegenüber der Inhalation in den Daten über detektierte biologische Schalldaten enthalten ist.

13. Verfahren gemäß Anspruch 11, bei dem die Schallquellenposition basierend auf der Expansion und Kontraktion von Lungen zur Zeit der Respiration eingestellt wird.

14. Verfahren zum Anzeigen eines medizinischen Bildes zum Anzeigen eines medizinischen Bildes (G), das zur Zeit der Bilddiagnose zu lesen ist, mit den folgenden Schritten:
einen Erfassungsschritt für Radiogramm-Bilddaten zum Erfassen der Radiogramm-Bilddaten (20, 60) eines vorbestimmten Teils eines mittels Radiogramm zu untersuchenden Objekts;
einen Detektierschritt für biologische Schalldaten (54) zum Erfassen der biologischen Schalldaten von dem Objekt;
einen Spezifizierschritt für die Schallquellenposition (55) zum Spezifizieren der Schallquellenposition, von der die biologischen Schalldaten detektiert werden; und
einen Bildanzeigeschritt (58) zum nebeneinander Anzeigen eines Schallquellenpositionsbildes (G11, G22), das sich auf die spezifizierte Schallquellenposition bezieht, und des Radiogramm-Bildes (G), das sich auf die Radiogramm-Bilddaten bezieht.

15. Verfahren gemäß Anspruch 14, bei dem mindestens die Radiogramm-Bilddaten und/oder biologischen Schalldaten über die Datenleitung erfasst werden.

16. Verfahren gemäß Anspruch 14, bei dem die Radiogramm-Bilddaten die Bilddaten sind, die durch mindestens eines der Folgenden erhalten werden: Röntgenstrahlen, CT, MRI, magnetische Instrumentierung, biologisches elektrisches Signal, Ultraschallwelle und Licht.

17. Verfahren gemäß Anspruch 14, bei dem die Schallquellenposition gemäß der Position des Mikrophons spezifiziert wird, wenn der biologische Schall gesammelt wird.

18. Verfahren gemäß Anspruch 14, bei dem der Detektierschritt für biologische Schalldaten, der Spezifierschritt für die Schallquellenposition und der Bildanzeigeschritt abwechselnd wiederholt werden, bis eine Anweisung zur Beendigung eingegeben wird.

19. Verfahren gemäß Anspruch 14, bei dem der Bildanzeigeschritt ferner die folgenden Schritte umfasst:
einen Bildextrahierschritt zum Extrahieren des Bildes eines vorbestimmten Bereichs, der die Schallquellenposition des Radiogramm-Bildes einschließt; und
einen Vergrößerungsschritt zum Vergrößern des extrahierten Bildes, um das extrahierte Bild in einer vergrößerten Form anzuzeigen.

20. Verfahren gemäß Anspruch 19, bei dem das extrahierte Bild in einer vergrößerten Form in einem getrennten Anzeige-Frame angezeigt wird.

21. Verfahren gemäß Anspruch 19, bei dem das extrahierte Bild in einer vergrößerten Form gemäß der Fläche des Radiogramm-Bildes ist, das die Schallquelle einschließt.

22. Verfahren gemäß Anspruch 14, bei dem der Bildanzeigeschritt ferner die folgenden Schritte umfasst:
einen Identifizierschritt zum Identifizieren des Typs des biologischen Schalls aus den detektierten biologischen Schalldaten, um die Schallquellenposition basierend auf der Form der Anzeige anzuzeigen, die dem identifizierten Typ des biologischen Schalls entspricht.

23. Verfahren gemäß Anspruch 19, bei dem die Schallquellenposition in dem extrahierten Bild in einer vergrößerten Form mit dem Typ der Schallquelle angezeigt wird.

24. Verfahren gemäß Anspruch 14, wenn die detektierten biologischen Schalldaten sich auf die Daten über den respiratorischen Schall beziehen, die Schallquellenposition des respiratorischen Schalls als Antwort auf entweder Exspiration oder Inhalation bei dem Bildanzeigeschritt eingestellt wird, und die Schallquellenposition im Anschluss zu dieser Einstellung angezeigt wird.

25. Verfahren gemäß Anspruch 24, bei dem die Information zum Identifizieren der Exspiration gegenüber der Inhalation in den Daten über detektierte biologische Schalldaten enthalten ist.

26. Verfahren gemäß Anspruch 24, bei dem die Schallquellenposition basierend auf der Expansion und Kontraktion von Lungen zur Zeit der Respiration eingestellt wird.

27. Computerprogramm, um einen Computer zu steuern, um als eine medizinische Bildanzeige zu arbeiten, wobei das Computerprogramm die medizinische Bildanzeige veranlasst, das Verfahren gemäß einem der Ansprüche 1 bis 26 durchzuführen.

28. Aufzeichnungsmedium, das ein Computerprogramm gemäß Anspruch 27 umfasst.

## Revendications

1. Procédé d'affichage d'images médicales, destiné à afficher une image médicale F(G) à lire lors d'un diagnostic sur image, comprenant les étapes suivantes :
une étape d'acquisition de données d'image radiographiées destinée à acquérir les données (20, 60) d'une partie prédéterminée d'un objet à radiographier ;
une étape de détection de données sonores biologiques (54) destinée à détecter les données sonores biologiques provenant de l'objet ;
une étape de définition de position de source sonore (55) destinée à définir la position de la source sonore d'où sont détectées les données sonores biologiques ; et
une étape d'affichage d'image (58) destinée à afficher l'image médicale comprenant une image de position de source sonore (G1, G2) associée à la position de source sonore définie, et l'image radiographiée (G) associée aux données d'image radiographiées, de telle sorte que l'image de position de source sonore est superposée à une partie de l'image radiographiée correspondant à la position de source sonore définie.

2. Procédé selon la revendication 1, dans lequel au moins une des données d'image radiographiées et les données sonores biologiques sont acquises à partir d'une base de données via une ligne de données.

3. Procédé selon la revendication 1, dans lequel les données d'image radiographiées sont les données d'image capturées par au moins un parmi des rayons X, un CT, un IRM, un instrument de mesure magnétique, un signal électrique biologique, une onde ultrasonique et la lumière.

4. Procédé selon la revendication 1, dans lequel la position de la source sonore est définie selon la position du microphone lors de la capture du son biologique.

5. Procédé selon la revendication 1, dans lequel l'étape de détection de données sonores biologiques, l'étape de définition de position de source sonore et l'étape d'affichage d'image sont répétées alternativement jusqu'à ce qu'une instruction de terminaison ait été saisie.

6. Procédé selon la revendication 1, dans lequel l'étape d'affichage d'image comprend en outre les étapes suivantes :
une étape d'extraction d'image destinée à extraire l'image d'une zone prédéterminée comprenant la position de la source sonore à partir de l'image radiographiée ; et
une étape d'agrandissement destinée à agrandir l'image extraite pour afficher l'image extraite sous une forme agrandie.

7. Procédé selon la revendication 6, dans lequel l'image extraite sous une forme agrandie est affichée dans un cadre d'affichage séparé.

8. Procédé selon la revendication 6, dans lequel l'image extraite sous une forme agrandie dépend de la surface de l'image radiographiée comprenant la source sonore.

9. Procédé selon la revendication 1, dans lequel l'étape d'affichage d'image comprend en outre les étapes suivantes :
une étape d'identification destinée à identifier le type du son biologique à partir des données sonores biologiques détectées pour afficher la position de la source sonore, en se basant sur la forme de l'affichage correspondant au type identifié du son biologique.

10. Procédé selon la revendication 6, dans lequel la position de la source sonore dans l'image extraite sous une forme agrandie est affichée avec le type de la source sonore.

11. Procédé selon la revendication 1, dans lequel les données sonores biologiques détectées sont associées aux données sur le son respiratoire, la position de la source sonore du son respiratoire est réglée en réponse soit à l'expiration, soit à l'inhalation, dans l'étape d'affichage d'image, et la position de la source sonore résultant de ce réglage est affichée.

12. Procédé selon la revendication 11, dans lequel les informations destinées à identifier l'expiration de l'inhalation sont comprises dans les données sur les données sonores biologiques détectées.

13. Procédé selon la revendication 11, dans lequel la position de la source sonore est réglée en se basant sur la dilatation et la contraction des poumons lors de la respiration.

14. Procédé d'affichage d'image médicale, destiné à afficher une image médicale (G) à lire lors du diagnostic d'image, comprenant les étapes suivantes :
une étape d'acquisition de données d'image radiographiées destinée à acquérir les données d'image radiographiées (20, 60) d'une partie prédéterminée d'un objet à radiographier ;
une étape de détection de données sonores biologiques (54) destinée à détecter les données sonores biologiques à partir de l'objet ;
une étape de définition de position de source sonore (55) destinée à définir la position de la source sonore d'où les données sonores biologiques sont détectées ; et
une étape d'affichage d'image (58) destinée à afficher une image de position de source sonore (G11, G22) associée à la position de la source sonore définie et l'image radiographiée (G) associée aux données d'image radiographiées, côte à côte.

15. Procédé selon la revendication 14, dans lequel au moins l'une des données d'image radiographiées et les données sonores biologiques sont acquises à partir d'une base de données via une ligne de données.

16. Procédé selon la revendication 14, dans lequel les données d'image radiographiées sont les données d'image capturées par au moins l'un parmi les rayons X, un CT, un IRM, un instrument de mesure magnétique, un signal électrique biologique, une onde ultrasonique et la lumière.

17. Procédé selon la revendication 14, dans lequel la position de la source sonore est définie selon la position du microphone lors de la capture du son biologique.

18. Procédé selon la revendication 14, dans lequel l'étape de détection des données sonores biologiques, l'étape de définition de position de source sonore et l'étape d'affichage d'image sont répétées alternativement jusqu'à ce qu'une instruction de terminaison ait été saisie.

19. Procédé selon la revendication 14, dans lequel l'étape d'affichage d'image comprend en outre les étapes suivantes :
une étape d'extraction d'image destinée à extraire l'image d'une surface prédéterminée comprenant la position de la source sonore dans l'image radiographiée ; et
une étape d'agrandissement destinée à agrandir l'image extraite pour afficher l'image extraite sous une forme agrandie.

20. Procédé selon la revendication 19, dans lequel l'image extraite sous une forme agrandie est affichée dans un cadre d'affichage séparé.

21. Procédé selon la revendication 19, dans lequel l'image extraite sous une forme agrandie dépend de la surface de l'image radiographiée comprenant la source sonore.

22. Procédé selon la revendication 14, dans lequel l'étape d'affichage d'image comprend en outre les étapes suivantes :
une étape d'identification destinée à identifier le type du son biologique à partir des données sonores biologiques détectées pour afficher la position de la source sonore en se basant sur la forme de l'affichage correspondant au type identifié du son biologique.

23. Procédé selon la revendication 19, dans lequel la position de la source sonore dans l'image extraite sous une forme agrandie est affichée avec le type de la source sonore.

24. Procédé selon la revendication 14, dans lequel lorsque les données sonores biologiques détectées sont associées aux données sur le son respiratoire, la position de la source sonore du son respiratoire est réglée en réponse, soit à l'expiration, soit à l'inhalation, dans l'étape d'affichage d'image, et la position de la source sonore résultant de ce réglage est affichée.

25. Procédé selon la revendication 24, dans lequel les informations d'identification de l'expiration par rapport à l'inhalation sont comprises dans les données sur les données sonores biologiques détectées.

26. Procédé selon la revendication 24, dans lequel la position de la source sonore est réglée en se basant sur la dilatation et la contraction des poumons lors de la respiration.

27. Programme informatique pour commander un ordinateur afin de fonctionner en tant qu'écran d'affichage d'image médicale, dans lequel le programme informatique amène l'écran d'affichage d'image médicale à exécuter le procédé selon l'une quelconque des revendications 1 à 26.

28. Support d'enregistrement, qui comprend un programme informatique selon la revendication 27.
